(19) Europäisches Patentamt / European Patent Office / Office européen des brevets

(11) **EP 4 285 862 A1**

(12) **EUROPEAN PATENT APPLICATION**
published in accordance with Art. 153(4) EPC

(43) Date of publication:
06.12.2023 Bulletin 2023/49

(21) Application number: 22746173.8

(22) Date of filing: 24.01.2022

(51) International Patent Classification (IPC):
*A61C 8/00* (2006.01)   *A61L 27/04* (2006.01)
*A61L 27/40* (2006.01)   *A61L 27/56* (2006.01)
*B33Y 80/00* (2015.01)   *B33Y 10/00* (2015.01)

(52) Cooperative Patent Classification (CPC):
A61C 8/00; A61L 27/04; A61L 27/40; A61L 27/56;
B33Y 10/00; B33Y 80/00

(86) International application number:
PCT/KR2022/001190

(87) International publication number:
WO 2022/164146 (04.08.2022 Gazette 2022/31)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 26.01.2021 KR 20210010770
21.01.2022 KR 20220009112

(71) Applicant: AON Co., Ltd.
Gyeonggi-do 15850 (KR)

(72) Inventors:
• KIM, Jong Kuk
  Hwaseong-si Gyeonggi-do 18320 (KR)
• KIM, Tack Yun
  Anyang-si Gyeonggi-do 14045 (KR)
• SO, Sung Min
  Gunpo-si Gyeonggi-do 15876 (KR)

(74) Representative: Frenkel, Matthias Alexander
Wuesthoff & Wuesthoff
Patentanwälte PartG mbB
Schweigerstraße 2
81541 München (DE)

(54) **ARTIFICIAL DENTAL ROOT, ARTIFICIAL BONE, AND MANUFACTURING METHOD THEREOF**

(57) The present specification discloses an artificial dental root, and a method for manufacturing the artificial dental root by 3D printing.

FIG. 1

Single-material experimental artificial dental root

$3Y-ZrO_2$
$ZrO_2-Al_2O_3$
$ZrO_2-HA$
$ZrO_2-TCP$
$ZrO_2-Al_2O_3-HA$
$ZrO_2-Al_2O_3-TCP$
etc.

EP 4 285 862 A1

## Description

### BACKGROUND

**Technical Field**

The present disclosure relates to an artificial dental root, an artificial bone, and a method for manufacturing the same.

**Background Art**

Ceramic materials are high value-added materials that are widely used in various fields such as structure, environment, and energy as well as biomedical fields such as medical implants, artificial bones, and artificial teeth. In general, ceramic powder is mixed with a liquid phase (polymer, etc.) to produce a ceramic slurry, paste, or dough form, and it is manufactured into a component material having a three-dimensional shape using various molding techniques.
Today, various ceramic-based 3D printing technologies that can manufacture ceramic materials are being developed. Specifically, there are FDM (Fused Deposition Modeling) method, in which heat-melting solid plastic material is drawn out like a thread and built up while being melted little by little, SLA (Stereo Lithography Apparatus) method using the principle of scanning a laser beam on a photocurable resin and curing the scanned part, SLS (Selective Laser Sintering) method using the principle of scanning a laser beam on functional polymer or metal powder used instead of photocurable resin in the SLA method and sintering and molding the scanned part, and DLP (Digital Light Processing) method using the principle of irradiating light to the bottom of a storage tank in which a photocurable resin is stored and partially curing the photocurable resin.
Among them, the photocuring 3D printing method is a method of manufacturing a complex shape through a lamination technology in which two-dimensional planes are laminated by selectively irradiating light in the UV or visible ray region to a composite containing a photocurable material, technology maturity of which is relatively low compared to other 3D printing technologies. This is because it is difficult to prepare highly filled photocurable ceramic slurry having a high ceramic content and flowability suitable for 3D printing in order to form a high-quality ceramic structure. In particular, as the ceramic content increases, the slurry viscosity rapidly increases to decrease the flowability. In addition, in the photocuring 3D printing method, when the content of a pigment increases while the two-dimensional planes are laminated, interlayer adhesion between layers of an object may be weakened, making it difficult to form the object.
In addition, since dental implant treatment surgically implants a screw-shaped titanium fixture after drilling an alveolar bone (gum bone), inflammation may occur in the alveolar bone and alveolar bone necrosis may occur.
Further, since a titanium-based alloy is used, problems such as low aesthetics, low biocompatibility, titanium elution, and hypersensitivity to titanium may occur. In order to solve such problems, in the field of titanium-based fixtures, surface roughness is controlled or ceramic is coated on the surface through a process to improve low biocompatibility and induce osteogenesis.

### SUMMARY

Rather than the current approach in the industry, it is necessary to improve the fundamental problem of the screw-shaped fixture configuration and titanium materials. In the present disclosure, a realistic ceramic artificial dental root is proposed as an alternative to solve the above problems.
In addition, the technical problems to be solved by the present disclosure are not limited to the above-mentioned technical problems, and other technical problems not mentioned above will become clear to those skilled in the art from the following descriptions.
An artificial dental root according to one embodiment of the present disclosure to solve the above problems may contain ceramic component and have a plurality of fluid channels formed therein. The artificial dental root may comprise an inner body and an outer body, and the fluid channels may be formed more in the outer body than in the inner body. The outer body may be formed of a plurality of layers, and the fluid channels may be formed more in an outermost outer body forming a surface of the artificial dental root than in a first external body in contact with the inner body. The ceramic component may include tetragonal zirconia, and further includes at least one of alumina, hydroxyapatite, and tricalcium phosphate. The artificial dental root may comprise an inner body and an outer body, and the outer body may include at least one of alumina, hydroxyapatite, and tricalcium phosphate more than the inner body.
Further, an artificial dental root according to another embodiment may contain a ceramic component and may comprise at least one of alumina, hydroxyapatite, and tricalcium phosphate. The artificial dental root may comprise an inner body and an outer body, and the outer body may include at least one of alumina, hydroxyapatite, and tricalcium phosphate more than the inner body.

Since the artificial dental root according to the present disclosure is made of a ceramic material, it exhibits excellent biocompatibility to alveolar bone compared to a titanium material and exhibits superior aesthetics compared to the titanium material.

In addition, since the artificial dental root according to the present disclosure can be naturally combined with the alveolar bone, there is no need to configure a screw line for coupling with the alveolar bone on the surface of the artificial dental root, and the artificial dental root is simply inserted into the inner cavity of the alveolar bone from which the existing dental root has been removed, thereby minimizing surgical invasion.

In addition, the present disclosure proposes a method for manufacturing the artificial dental root exhibiting the above effects with a 3D printer.

## BRIEF DESCRIPTION OF THE DRAWINGS

FIGS. 1 to 6B are diagrams for explaining individual embodiments of an artificial dental root.
FIGS. 7 to 10 are diagrams for explaining individual embodiments of a 3D printing method for manufacturing the artificial dental root.

## DESCRIPTION OF EXEMPLARY EMBODIMENTS

Terms used in the present specification are only used to describe the specific embodiments, and are not intended to limit the present disclosure. Singular expressions include plural expressions unless the context clearly indicates otherwise. In the present specification, it should be understood that the terms "comprise", "include" or "have" are intended to designate that there is a feature, a step, a component, or a combination thereof, but the possibility of presence or addition of one or more other features, steps, components, or combinations thereof is not precluded.

Further, in the present disclosure, when each layer or element is referred to as being formed "on" or "above" respective layers or elements, it means that each layer or element is formed directly on the respective layers or elements, or that another layer or element may be additionally formed between the respective layers, or on the object or substrate.

Since the present disclosure may be variously modified and have various embodiments, specific embodiments are illustrated in the drawings and described in detail below. However, it should be understood that this is not intended to limit the present disclosure to the specific embodiments, and that the present disclosure includes all modifications, equivalents, and substitutes included in the idea and scope of the present disclosure.

In the present specification, a ceramic material applicable to an artificial dental root and artificial bone and a method for generating the artificial dental root and artificial bone using the ceramic material are disclosed. For example, the disclosure herein may be used to manufacture artificial dental roots for dental implant treatment. Hereinafter, the artificial dental root will be described as an example, but the following description can also be applied to the artificial bone.

In one embodiment, the artificial dental root may be made of a ceramic material. In one embodiment, 3D shape data may be obtained from an actual natural dental root of a patient in need of dental implant treatment, and an individually customized ceramic artificial dental root for the corresponding dental root may be manufactured using a 3D printing method.

In addition, the realistic ceramic artificial dental root may be formed of a plurality of layers. For example, the realistic ceramic artificial dental root may include an inner body having bending strength that can withstand external stress, and an outer body having pores and/or channels that are excellent in bonding with a human alveolar bone and are advantageous for bone formation in order to be bonded to the alveolar bone after being implanted in the alveolar bone. In this case, the pores and the channels may form fluid channels. The inner body and the outer body may be made of different materials, or may have different densities or mixing ratios of materials even if they are made of the same material. For example, the artificial root may be made in a gradient structure so that the inner body is denser than the outer body. Hereinafter, the artificial dental root according to the present disclosure will be described by way of example.

## Embodiment 1

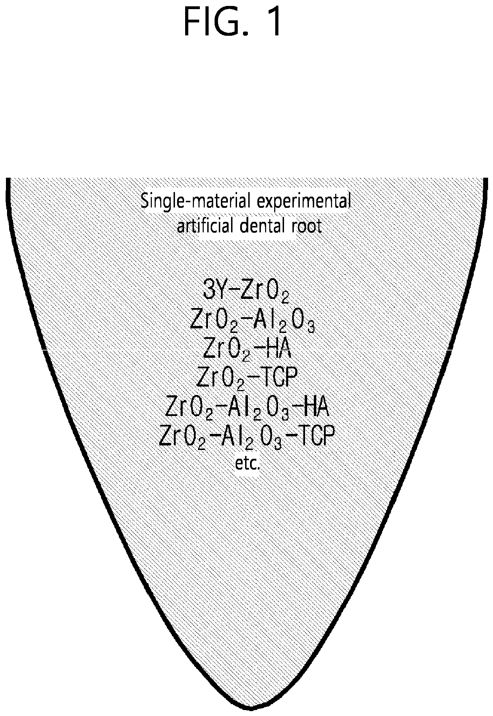

FIG. 1 is a diagram showing the configuration of an artificial dental root according to one embodiment. In one embodiment, the artificial dental root may be densely formed of a single material. For example, the artificial dental root may be made of a single material of tetragonal zirconia ($ZrO_2$) ($3Y-ZrO_2$). The tetragonal zirconia referred to herein and in the description below may contain yttria. For example, the tetragonal zirconia may be partially stabilized $ZrO_2$ ($3Y-ZrO_2$) containing 3 mol% yttria.

Alternatively, the artificial dental root may be formed of a tetragonal zirconia composite containing at least one

of alumina (Al$_2$O$_3$), hydroxyapatite (HAP) and/or tricalcium phosphate (TCP). For example, the ceramic artificial dental root may be formed of tetragonal zirconia alone, or a tetragonal zirconia composite such as a tetragonal zirconia-alumina composite, a tetragonal zirconia-TCP composite, a tetragonal zirconia-HAP composite, a tetragonal zirconia-alumina-TCP composite, a tetragonal zirconia-alumina-HAP composite, a tetragonal zirconia-TCP-HAP composite, and/or a tetragonal zirconia-alumina-TCP-HAP composite.

Zirconia and alumina have excellent mechanical properties, have little or no foreign body reaction when implanted into a human body, and have superior mechanical properties compared to bioactive ceramic materials, and they are used for load-bearing artificial joints and prosthetic treatments, so they are materials that ensure stability. The tetragonal zirconia-alumina composite has superior fracture toughness and low specific gravity compared to tetragonal zirconia. TCP and HA are bioactive bioceramics, which are compounded with bioinert bioceramics ZrO$_2$ and Al$_2$O$_3$ to allow the artificial dental root contact the alveolar bone to form a strong bond therewith after the artificial dental root is implanted in the alveolar bone.

When the artificial dental root is made of a single material, the minimum relative density of the material constituting the artificial dental root may be limited to be 97%. Alternatively, the relative density of the material may be limited so that the bending strength of the artificial dental root has a value between 200 MPa and 1200 MPa.

In this case, the relative density of 97% or more may mean the relative density of the constituent material (irrelevant even if different materials are included). Here, the relative density of 97% means that a measured density is 3% lower than a theoretical density. In ceramics, when the density is low, the mechanical properties are deteriorated, so a high relative density is advantageous in resisting external stress. Here, the relative density may be calculated by dividing the measured density of the material by the theoretical density of the material. Here, the theoretical density is a theoretical density of a material (inherent property of the material). Here, the measured density may be an apparent density or a bulk density.

Meanwhile, the artificial dental root may be manufactured to have a porous structure or a channel structure. The porous structure and/or the channel structure of the artificial dental root surface are filled with osteogenic substances flowing from the alveolar bone, thereby helping the artificial dental root in biocompatibility with the alveolar bone and in bone formation therebetween.

To this end, for example, the porous structure may be formed in the surface of the artificial dental root for osteogenesis and bonding at a portion in contact with alveolar bone. The porous structure of the surface may serve as pores. In addition, a channel structure may be formed by continuously forming such pores from the surface of the artificial dental root inwardly. For example, the porous structure may form a channel structure so that the osteogenic substances can flow into the artificial dental root. For example, a channel structure such as a scaffold structure or a gyroid structure may be formed in the artificial dental root. The application of the channel structure can be implemented by performing 3D modeling of an artificial dental root 3D model as the scaffold structure or the gyroid structure. In the case of a single material and a single structure, the porous structure may be formed to the same extent on the entire artificial dental root, not just the surface, in order to implement pores/channels in the artificial dental root.

### Embodiment 2

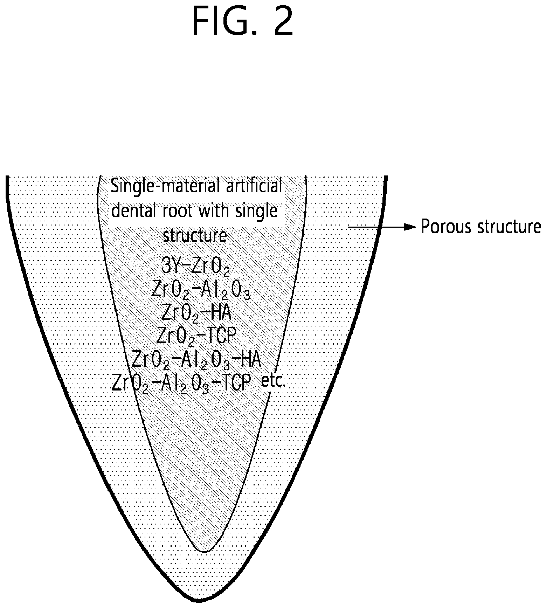

FIG. 2 is a diagram showing the configuration of an artificial dental root according to another embodiment. The artificial dental root may be formed of a plurality of layers. For example, the artificial dental root may include an inner body having bending strength that can withstand external stress, and an outer body having pores and/or channels that are excellent in bonding with a human alveolar bone and are advantageous for bone formation in order to be bonded to the alveolar bone after being implanted in the alveolar bone.

For example, the pores or the channels may not be formed in the inner body, but the pores or the channels may be formed in the outer body. To this end, even if the outer body is formed of the same material, the density or mixing ratio of the material may be varied. For example, the artificial dental root may be made in a gradient structure so that the inner body is denser than the outer body. For example, the minimum relative density of the inner body may be limited to be 97%. Alternatively, the relative density of the material may be limited so that a bending strength of the inner body has a value between 200 MPa and 1200 MPa. The relative density of the outer body may have a lower value than that of the inner body. Alternatively, the bending strength of the outer body may be lower than that of the inner body. For example, the inner body may have a porosity of 0.1% or less in order to show high strength while using the material in Embodiment 1. In addition, a first outer body in contact with the inner body may use the same material as the inner body, but may have a porosity of 5%. In addition, a second outer body in contact with the first outer body may use the same material as the inner body, but may have a porosity of 10%. In this way, the porosity of the outer body can be gradually increased by 5% as it goes from the inside to the outer surface. In one embodiment, the porosity of the outermost outer body in contact with the surface may be limited to 30%. In this case, the gradual increase rate of 5% is only an example, and the gradual increase rate may be selected and used as a predetermined percentage.

The area of the inner body may occupy a minimum of 50% (e.g. when the outer body is dominant) and a maximum of 100% (e.g. when the outer body is not present) based on the cross-section of the artificial dental root. If the area of the inner body is less than 50% of the cross-section of the artificial dental root, cracks may occur due to stress generated during the manufacturing. If the area of the inner body is 50% of the cross-section of the artificial dental root, the porous structure of the outer body may be applied up to 50% of the cross-section of the artificial dental root.

**Embodiment 3**

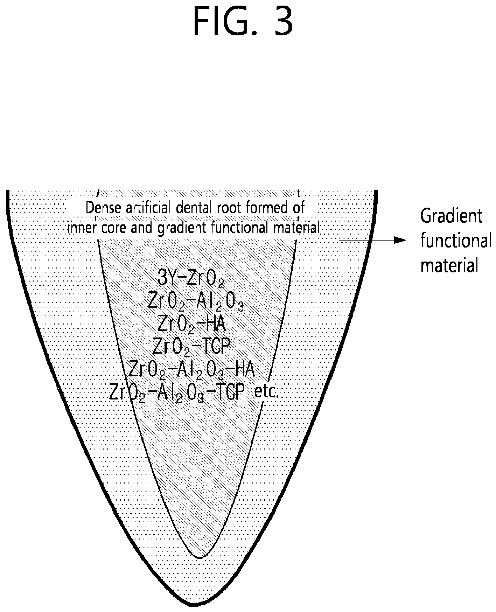

FIG. 3 is a diagram showing the configuration of an artificial dental root according to another embodiment. The artificial dental root according to one embodiment is formed of a plurality of layers of an inner body and an outer body, but may be formed of a gradient functional material rather than a porous structure in order to ensure biocompatibility.

For example, the inner body may be formed of tetragonal zirconia and/or a zirconia composite (e.g., a composite containing alumina, HAP, TCP, etc. described above). However, the outer body may be formed of a zirconia composite containing HAP or TCP.

In addition, the content of HAP or TCP in the inner body may be limited to a predetermined content in order to achieve bending strength. In addition, the content of HAP or TCP in the outer body may be limited to a predetermined content or more for bonding to the alveolar bone.

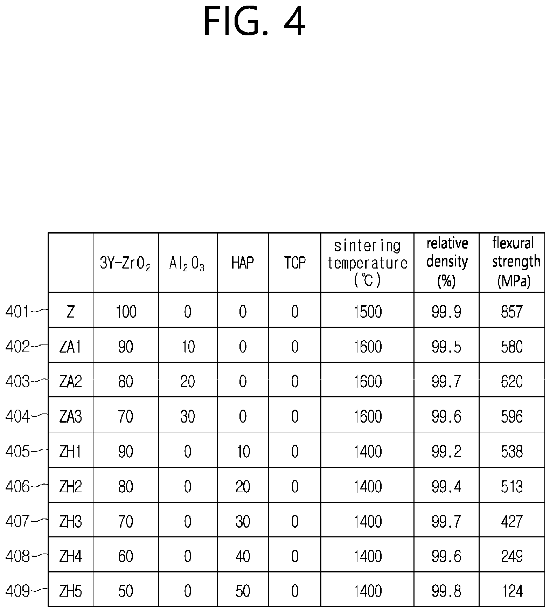

In one embodiment, the content of HAP in the inner body may be limited to 10 wt% or less (or less than 10 wt%). In addition, the content of HAP in the external body may be limited to exceed 10 wt% (or to 10 wt% or more). Furthermore, the artificial dental root may be manufactured so that the HAP content in an outer portion of the outer body constituting the surface of the artificial dental root is higher than the HAP content in an inner portion of the outer body facing the inner body to realize a predetermined gradient function. Referring to FIG. 4, the inner body is formed of Z material 401, a first outer body in contact with the inner body is formed of ZA1 material 402, and a second outer body in contact with the first outer body is ZA2 material 403, a third outer body in contact with the second outer body and constituting the surface material may be formed of ZA3 material 404.

When TCP is used as the gradient material, in the same way, the inner body is formed of ZH1 material 405, the first outer body in contact with the inner body is formed of ZH2 material 406, and the second outer body in contact with the first outer body is formed of ZH3 material 407, and the third outer body in contact with the second outer body and constituting the surface material may be formed of material ZH4 material 408.

Meanwhile, as shown in FIG. 4, in order to limit the appropriate bending strength to 224 MPa or more, the maximum content of HAP and/or TCP may be limited to no more than 40 wt%. For example, the maximum content of HAP and/or TCP in the outer body in the surface portion may be limited to no more than 40 wt%. Alternatively, the maximum content of HAP and/or TCP may be limited to the content for limiting the appropriate flexural strength (e.g., bending strength) to 200 MPa or more. In one embodiment, the maximum content of HAP and/or TCP may be limited to no more than 40 wt% in order to limit the appropriate bending strength to 200 MPa or more. In another embodiment, the maximum content of HAP and / or TCP may be limited in order to limit the appropriate flexural strength to 300 MPa or more. In another embodiment, the maximum content of HAP and/or TCP may be limited to no more than 30 wt% in order to limit the appropriate flexural strength to 400 MPa or more.

In addition, when alumina is used, the content of alumina in the inner body may be at least 0wt%, in which case the inner body may be formed of tetragonal zirconia only. Further, the maximum alumina content in the outer body may be 50wt%. In addition, the alumina contents in the layers of the outer body may be formed in a gradient structure so that the alumina content in the layer of the outer body close to the surface (e.g., the outermost outer body) is highest.

When a gradient material is used together with alumina, the contents of the alumina and the gradient material may be applied according to the previous description. For example, in the case of a tetragonal zirconia-alumina-HAP composite, 0 to 50 wt% of alumina and 0 to 30 wt% of HAP may be used. For example, the inner body may be formed of 100 wt% of tetragonal zirconia, 0 wt% of alumina, and 0 wt% of HAP. In addition, the first outer body may be formed of 80 wt% of tetragonal zirconia, 10 wt% of alumina, and 10 wt% of HAP. Further, the outermost outer body may be formed of 20 wt% of tetragonal zirconia, 50 wt% of alumina, and 30 wt% of HAP.

As another example, in the case of a tetragonal zirconia-alumina-TCP composite, 0 to 50 wt% of alumina and 0 to 30 wt% of TCP may be used. For example, the inner body may be formed of 100 wt% of tetragonal zirconia, 0 wt% of alumina, and 0 wt% of TCP. In addition, the first outer body may be formed of 80 wt% of tetragonal zirconia, 10 wt% of alumina, and 10 wt% of TCP. Further, the outermost outer body may be formed of 20 wt% of tetragonal zirconia, 50 wt% of alumina, and 30 wt% of TCP.

**Embodiment 4**

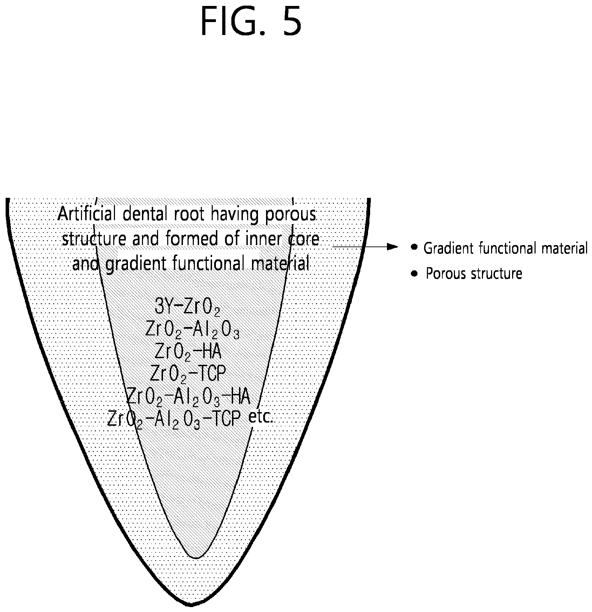

FIG. 5 is a diagram showing an artificial dental root according to another embodiment. The artificial dental root according to one embodiment is formed of a plurality of layers of an inner body and an outer body, and the outer body may be composed of a functional gradient material having a porous structure to ensure biocompatibility.

For example, as in Embodiment 3, the inner body may be formed of tetragonal zirconia and/or a zirconia composite (e.g., a composite containing alumina, HAP, TCP, etc. described above). However, the outer body may be formed of a zirconia composite containing HAP or TCP. In addition, the artificial dental root according to the present embodiment may be manufactured to include the porous structure in the outer body as in Embodiment 2.

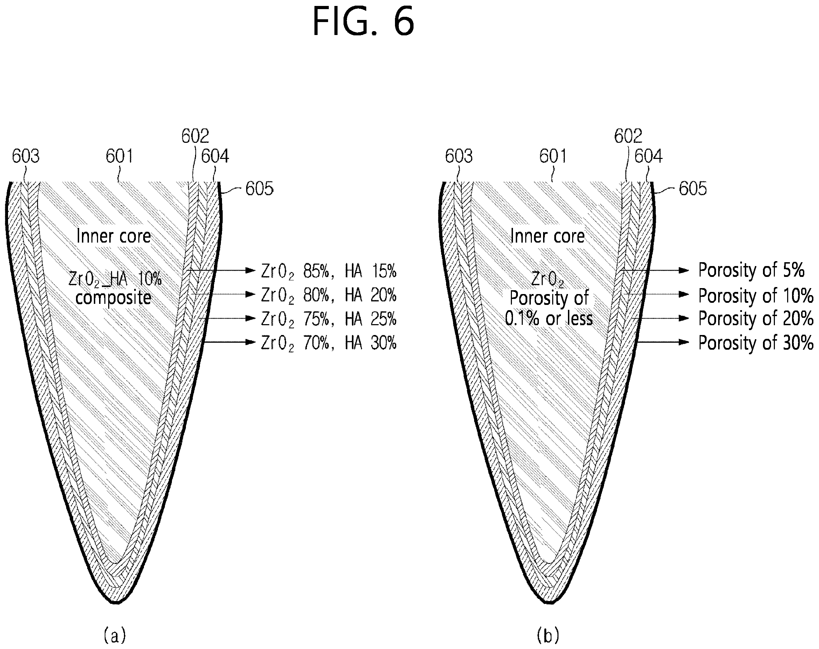

An example of this is shown in FIGS. 6A and 6B. The gradient structure of the artificial dental root depending on the material composition is shown in FIG. 6A, which shows an example of the artificial dental root according to Embodiment 3. Further, the gradient structure of the artificial dental root depending on the porosity is shown in FIG. 6B, which shows an example of the artificial dental root according to Embodiment 2. Meanwhile, the artificial dental root showing the characteristics of FIGS. 6A and 6B may be an artificial dental root model in which as an example of Embodiment 4, HAP is adopted as the gradient structure and porosity is applied as the gradient structure.

**Manufacturing method**

As disclosed above, the artificial dental root according to the present disclosure has a predetermined internal structure in the root. In this respect, it is difficult to manufacture a realistic artificial dental root made of ceramic material using a conventional mold or a cutting method.

Therefore, ceramic 3D printing technology may be applied to manufacture a personalized realistic artificial dental root. To this end, the shape and structure of the artificial dental root surface may be controlled through 3D modeling.

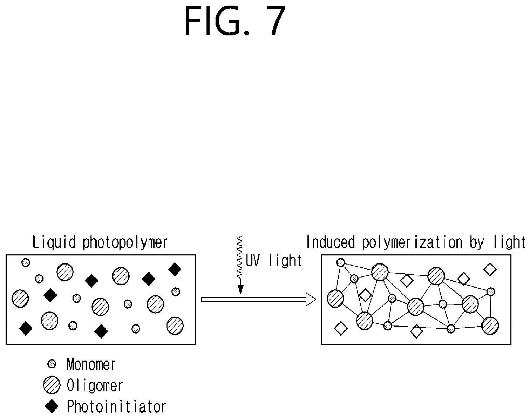

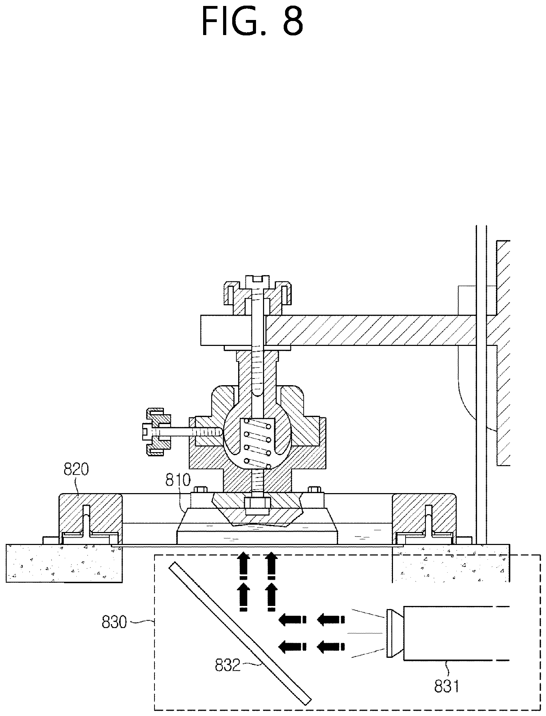

In one embodiment, an artificial dental root identical to a real dental root shape of a person can be manufactured by a 3D printing method. The basic concept for 3D printing of ceramic slurry materials is illustrated in FIGS. 7 to 9. FIG. 7 discloses the principle of crosslinking between monomer and oligomer by a photoinitiator. FIG. 8 shows a conceptual diagram of the operation of a 3D printer that performs ceramic 3D printing utilizing this concept.

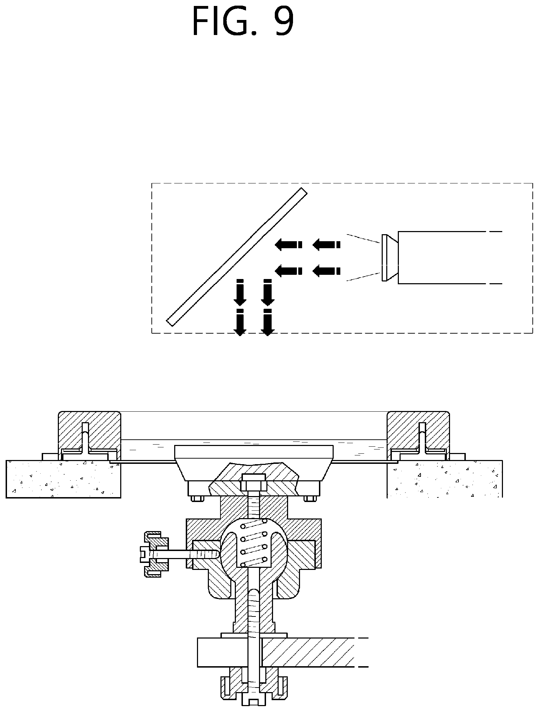

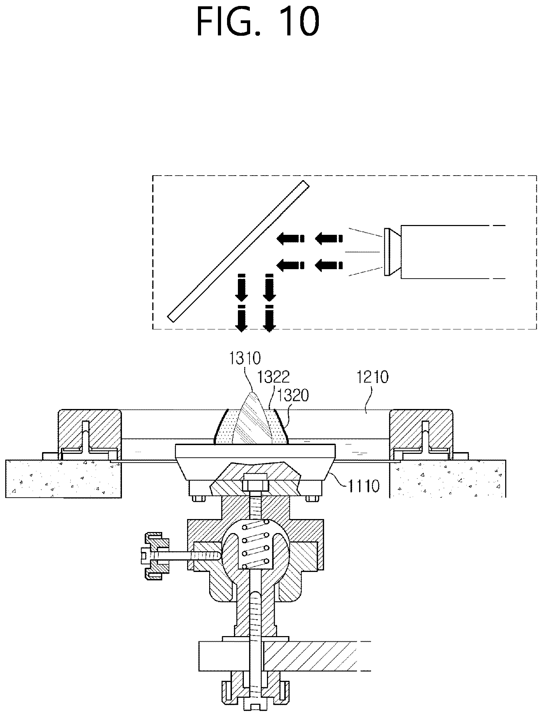

The 3D printer of FIG. 8 may include a stage 810, a tank 820, and a light irradiation unit 830. Ceramic slurry may be accommodated in the tank 820. The stage 810 descends so that a space between the bottom of the tank 820 and a lower surface of the stage 810 forms a space (e.g. a space forming one ceramic slide, which is one unit of performing 3D printing). The ceramic slurry remaining in the space is cured by the curing light irradiated to the water tank 820 through a light source 831 and a reflector 832. As a result, a ceramic slide of one unit is formed, and 3D printing may be performed by repeating this process a number of times. FIG. 8 shows a bottom-up 3D printing in which 3D printed objects are stacked downward on the lower surface of the stage, but a top-down 3D printing in which the stage is submerged into the tank while the slide is deposited on the stage as shown in FIG. 9 may be applied. When the top-down 3D printing is applied, as shown in FIG. 10, there is an advantage in that an outer body can be additionally added to a pre-shaped artificial dental root. FIG. 10 illustrates an example in which an outer body 1320 is stacked on an outer side of an inner body 1310 in a top-down manner. As shown in FIG. 10, a pre-formed internal body 1310 may be positioned on the stage 1110. As the stage 1110 is lowered to the bottom surface of the tank, the lower surface of the inner body 1310 may be submerged in a slurry 1210 in the tank. As the curing light is irradiated to a slurry surface 1322 corresponding to the outer body 1320 while the inner body 1310 is submerged from the bottom, one ceramic slide, which is one unit of printing for the outer body 1320, can be created. By repeating the lowering of the stage 1110 and the curing of the surface of the slurry 1322 corresponding to the outer body 1320, the outer body 1320 in contact with the inner body 1310 can be created using the 3D printing method.

Meanwhile, the above-described "slurry" generally means a liquid state with low fluidity containing a high concentration of suspended matter, and should be understood as including paste and dough states.

First, the ceramic slurry composition according to one embodiment of the present disclosure may include at least one of a ceramic powder, a photocurable binder, a dispersant, and a photocuring initiator.

The ceramic powder is a main material when manufacturing a structure using 3D printing. As described in Embodiments 1 to 4, the ceramic according to one embodiment of the present disclosure may be a tetragonal zirconia composite including at least one of tetragonal zirconia and/or alumina ($Al_2O_3$), hydroxyapatite (HAP), and/or tricalcium phosphate (TCP). Meanwhile, in the present disclosure, the ceramic powder may be included in an amount of 60 to 90 wt% based on the total weight of the slurry composition.

In addition, the zirconia powder may be stabilized with 2 to 8 mol% of yttria (Y), specifically 3 to 5 mol% of yttria (Y), based on the total composition of the zirconia powder. When the zirconia powder is stabilized with yttria in the above mol% range, zirconia powder or the like can be prepared in the form of granules, and in the case of such granules, a relatively large amount of ceramic components can be contained in the slurry composition, which increases the density during lamination. However, as the amount of yttria (Y) used for stabilization increases, there may be a problem in that the flexural strength of the finally manufactured output product decreases, the yttria content in the zirconia powder is preferably in the range of 2-8 mol%..

The photocurable binder serves to form an adhesive force between the ceramic powders to help form a molded body and a structure, and may be a mixture of a photocurable monomer and a photocurable oligomer.

Specifically, when only a low-shrinkage monomer is included as a photocurable binder, metal adhesion is weak while film adhesion is strong, and in the case of using SLA/DLP-type 3D printing, which will be described later, after the creation of a cured layer, in the process of separating the cured layer and the film at the same time as the platform is raised, the film may remain in the cured layer, and problems may occur in the manufacturing process of molded bodies and structures, such as problems in the post-processing after molding due to weak brittleness. In the case of using only an oligomer, due to viscosity of the oligomer itself, it is difficult to contain a high content of ceramic powder, and it may be difficult to increase the packing density.

Accordingly, the photocurable binder according to one embodiment of the present disclosure may be a mixture of a ratio of 8 to 25 parts by weight of photocurable monomer and 2 to 15 parts by weight of photocurable oligomer, and more specifically, a mixture of a ratio of 10 to 16 parts by weight of photocurable monomer and 3 to 9 parts by weight of photocurable oligomer. The composition having the above ratio of part by weight provides an appropriate level of bonding between the monomer and oligomer, thereby enabling a high level of filling density while preventing the cured layer from remaining in the film even when platform is raised after the cured layer is created in the SLA/DLP method of 3D printing.

Furthermore, in the present disclosure, the photocurable binder may include two or less functional groups. Specifically, in the monomer and oligomer constituting the photocurable binder, the photocurable binder having one functional group generally forms a linear bond between the adjacent monomer and oligomer, and the photocurable binder having two functional groups generally form a branched bond. Meanwhile, the photocurable binder having three or four functional groups forms a cross-linked bond, and the photocurable binder having five or more functional groups forms a network bond.

For example, in the slurry composition of the present disclosure, when the monomer and oligomer constituting the photocurable binder have three or more functional groups, the cross-linked bond or network bond is formed, resulting in a significantly higher bonding density between the monomer and oligomer. In particular, when cured and solidified by UV irradiation, shrinkage may occur, resulting in poor interlayer adhesion.

Accordingly, the photocurable binder used in the present disclosure may include at least one of the photocurable monomer and the photocurable oligomer. In this case, at least one of the photocurable monomer and the photocurable oligomer may include two or less functional groups. For example, the photocurable binder according to one embodiment may include a bifunctional monomer and a bifunctional oligomer, may include a monofunctional monomer and a bifunctional oligomer, may include a bifunctional monomer and a monofunctional oligomer, or may include a monofunctional monomer and a monofunctional oligomer. Accordingly, by minimizing the photocuring shrinkage rate under UV irradiation, the final manufactured product can exhibit excellent flexural strength, improve interlayer adhesion, and ensure structural stability of the final manufactured product.

More specifically, the photocurable monomer may be at least one selected from a monofunctional monomer and a bifunctional monomer.

For example, in the case of the monofunctional monomer, the photocurable monomer may be at least one or more selected from the group consisting of Stearyl Acrylate, Tetrahydrofufuryl Acrylate, Lauryl Acrylate, Ethoxylate(n) Nonyl Phenol Acrylate, Isodecyl Acrylate, Cycloaliphatic Acrylate, Methoxy polyethylene glycol monoacrylate, Alkoxylated phenol Acrylate, Triethylene glycol ethyl ether Methacrylate, Carprolactone Acrylate, Polypropylene glycol Monomethacrylate, Cyclic trimethylolpropane formal Acrylate, Phenoxy benzyl Acrylate, 3,3,5-Trimethyl cyclohexyl Acrylate, Isobornyl Acrylate, Isobornyl Metacrylate, 4-tert-butylcyclohexyl acrylate, Benzyl Acrylate, Biphenylmethyl Acrylate, Phenol (EO)n Acrylate, Phenoxyethyl Methacrylate, and N,N-Dimethyl acrylamide.

In another example of the bifunctional monomer, the photocurable monomer may be at least one or more selected from the group consisting of 1,6-Hexanediol diacrylate, 1,6-Hexanediol dimethacrylate, 1,6-Hexanediol (EO)n Diacrylate, Alkoylated hexanediol diacrylate, 1,4-Butanediol Dimethacrylate, Bisphenol A (EO)n Diacrylate, Cyclohexane dimethanol diacrylate, Ethoxylated bisphenol A dimethacrylated, Diethylene glycol diacrylate, Tripropyleneglycol diacrylate, Neopentyl glycol diacrylate, Dipropylene glycol diacrylate, Propoxylated(2) neopentyl glycol diacrylate, Tricyclodecane dimethanol diacrylate, 1,3 Butylene glycol dimethacrylate, Hydroxy pivalic acid neopentyl glycol Diacrylate, Neopentylglycol (PO)n Diacrylate, Ethylene glycol Dimethacrylate, Triethylene glycol Diacrylate, and Triethylene glycol Dimethacrylate.

The photocurable oligomer may include an oligomers having two or less functional groups. For example, the photocurable oligomer may include one or more oligomers selected from the group consisting of urethane oligomer (e.g., urethane acrylate), oligomer containing a phospheric acid group (e.g., phospheric acid-modified acrylate), oligomer containing a carboxylic acid group (e.g., carboxylic acid-modified acrylate), and epoxy oligomer (e.g., epoxy acrylate). The Oligomer can provide high adhesion to a platform (printing bed) used as a metallic material, particularly in 3D printing in which the SLA/DLP method is mixed. Meanwhile, when only the urethane oligomer is used as the photocurable oligomer, adhesion to metal may be poor, causing film to remain in the cured layer during the process of separating the cured layer from the film. Accordingly, when the urethane oligomer is used, the binder may further include an oligomer including an acidic group with high metal adhesion or an epoxy oligomer. Here, the oligomer including an acidic group may be an oligomer including a phospheric acid group (e.g., phospheric acid-modified acrylate) or an oligomer including g a carboxylic acid group (e.g., carboxylic acid-modified acrylate).

Meanwhile, in one embodiment of the present disclosure, the photocurable binder including the photocurable monomer and the photocurable oligomer may be 10 to 40 wt% based on the total weight of the composition.

The dispersant serves to prevent the dispersion and re-aggregation of the ceramic powder (filler) so that the ceramic powder can be sufficiently included in the product, and the dispersant common in the art may be selected and used, and may include, for example, one or more selected from the group consisting of copolymer compounds having acidic groups and polyester/polyether-based compounds having phospheric acid groups and amine groups. Meanwhile, in one embodiment of the present disclosure, the dispersant used may be from 1 to 5 wt% based on the total weight of the composition.

The photoinitiator serves to absorb light during UV or LED irradiation and emit radicals, enabling the bonding of the monomer and oligomer to form a solid polymer, and since an LED can be used as a light source, especially in 3D printers, the photoinitiator with a wavelength range of 370 to 420 nm may also be used.

Meanwhile, in the present disclosure, when the slurry composition is for depositing a thin film with a thickness of 15 $\mu$m or less per layer (e.g., 10 to 15 $\mu$m), the photocuring initiator may include one or more selected from a short wavelength initiator having a wavelength of less than 360 nm, e.g., a wavelength of 250 to 360 nm, a medium wavelength initiator, or a mixture thereof.

The short or medium wavelength initiator according to one embodiment of the present disclosure may be one or more selected from the group consisting of 1-hydroxycyclohexyl phenyl ketone, Benzophenone, 4-Methylbenzophenone, 4-Benzoyl-4'-Methyldiphenylsulfide, Methyl phenylglyoxylate, Methyl o-benzoylbenzoate, Benzil dimethyl ketal, 4-phenylbenzophenone, 2-Ethylhexyl-4-Dimethylaminobenzoate, Ethyl-4-Dimethylaminobenzoate, Hydroxy-2-methylphenyl-propane-1-one, 2-Benzyl-2-(dimethylamino)-1-[4-(morpholinyl)phenyl]-1-butanone, 2-Dimethylamino-2-(4-methylbenzyl)-1-(4-morpholin-4-yl-phenyl)butan-1-one, Oligo[2-hydroxy-2-methyl-1-[4-(1-methylvinyl)phenyl] propanone], and 2-hydroxy-2-methyl-1-phenyl propan-1-one.

Meanwhile, in the present disclosure, when the slurry composition is for depositing a thin film (thick film) with a thickness greater than 15 $\mu$m per layer (e.g., 20 $\mu$m), the photocuring initiator may include a long-wavelength initiator having a wavelength of 360 nm or more, specifically 360 to 450 nm.

The long-wavelength initiator according to one embodiment of the present disclosure may be one or more selected from the group consisting of Isopropylthioxanthone, 4,4'-Bis(diethylamino)benzophenone, 2,4-diethylthioxanthone, 2,4,6-Trimethylbenzoyl-diphenyl phosphine oxide, Phosphine oxide, Bis(2,4,6-trimethylbenzoyl)-phenylphosphineoxide; Bis-(eta 5-2,4,-cyclopentadien-1-yl)-Bis[2,6-difluoro-3-(1H-pyrrol-1-yl)-phenyl]titanium.

Meanwhile, when the type of initiator is changed depending on the thickness of the thin film (or thick film) as described above, the control of the curing rate and curing ability according to the thickness is optimized, which enables excellent interlayer adhesion to be secured. The photocuring initiator according to one embodiment of the present disclosure may be 0.01 to 1 wt% based on the total weight of the composition.

The ceramic slurry composition according to the present disclosure, as described above, can increase ceramic filling density in 3D printing, especially in 3D printing in which the SLA/DLP method is mixed, while minimizing photocuring shrinkage to thereby achieve excellent flexural strength in the range of 200 to 1,000 MPa, improve interlayer adhesion, and secure structural stability.

In particular, the ceramic slurry composition according to the present disclosure uses photocurable monomer and oligomer including two or less functional groups as the photocurable binder by optimizing the composition and type thereof, so that it is easy to achieve proper flowability, high interlayer adhesion, high flexural strength of 500 MPa or more, and excellent structural stability, especially in 3D printed outputs with the SLA/DLP method mixed.

The operation and effectiveness of the present disclosure will be described in more detail through specific embodiments of the present disclosure. However, it is presented as an example of the present disclosure and does not limit the scope of the present disclosure in any way.

## Examples 1 to 19

Ceramic slurry compositions were prepared as follows with the ingredients and composition ratios as shown in Tables 1 to 4 below. Specifically, the monomer was mixed with a photoinitiator and stirred for 2 hours at 300 rpm or more in a stirrer to completely dissolve the photoinitiator, and a binder (oligomer and/or dispersant) was mixed therewith and stirred for 30 minutes at 200 rpm or more. Finally, the ceramic slurry composition was prepared by mixing the ceramic powder therein and stirring for 10 minutes at 1,500 rpm using a paste mixer.

Meanwhile, the ceramic slurry composition was laminated by a 3D printing method in which the SLA/DLP method is mixed to form a 3D structure. In one aspect, as shown in FIG. 8, the SLA/DLP method may perform the following process using a 3D printer including a printing bed; a tank unit having at least one tank located below the printing bed; a tank moving unit for moving the tank; a light irradiation unit located below the tank; and a control unit for controlling the tank moving unit to move the tank (see FIG. 8).

STEP 1: A shallow slurry layer is formed on the film in the tank. In this case, the distance between the platform (printing bed) and the tank is sufficient to create a slurry layer. STEP 2: In order to cure one laminate, the platform is lowered to create a gap of about 1 mm between the platform and the film, and the slurry in STEP 1 is positioned between the platform and the film. STEP 3: By irradiating UV light that penetrates the film from the bottom of the film to cure the slurry, one curing layer is formed. STEP 4: After forming one cured layer, the platform is raised upwards and the cured layer is separated from the film. STEP 5: Repeating STEPS 1 to 4.

(Table 1)

| | Example 1 | Example 2 | Example 3 | Example 4 | Example 5 | Example 6 | Example 7 |
|---|---|---|---|---|---|---|---|
| 3 mol% yttria-stabilized zirconia | 85 | 75 | 60 | | | 50 | 50 |
| 8 mol% yttria-stabilized zirconia | | | | 75 | | | |
| Alumina | | | | | 75 | 25 | |
| Silica | | | | | | | 25 |
| Long-wavelength initiator: omnirad 819(Bis(2,4,6-trimethylbenzoyl)-phenylphosphineoxide) | 0.2 | 0.3 | 0.5 | 0.3 | 0.3 | 0.3 | 0.3 |
| THFA(Tetrahydrofufuryl Acrylate monofunctional monomer) | 8.8 | 14.8 | 23.6 | 14.8 | 14.8 | 14.8 | 14.8 |
| Urethane Acrylate | 3.5 | 5.8 | 9.3 | 5.8 | 5.8 | 5.8 | 5.8 |
| Phosphoric acid-modified acrylate | 1.5 | 2.5 | 4 | 2.5 | 2.5 | 2.5 | 2.5 |
| Dispersant | 1.0 | 1.6 | 2.6 | 1.6 | 1.6 | 1.6 | 1.6 |
| Flexural Strength (MPa) | 780 | 510 | 230 | 170 | 340 | 390 | 240 |

The results shown in Table 1 above are examples prepared by using different types of ceramic types or varying the content of ceramic, and it was confirmed that flexural strength of 200 MPa or more was secured in all of the Examples except for Example 4.

(Table 2)

| | Example 2 | Example 8 | Example 9 | Example 10 | Example 11 |
|---|---|---|---|---|---|
| 3 mol% Yttria-stabilized zirconia | 75 | 75 | 75 | 75 | 75 |
| Long-wavelength initiator: omnirad 819 | 0.3 | 0.02 | 1.0 | | 1.5 |
| Short wavelength initiator: omnirad 184 (1-hydroxycyclohexyl phenyl ketone) | | | | 0.3 | 1.5 |
| THFA | 14.8 | 15.08 | 14.1 | 14.8 | 14.8 |
| Urethane acrylate | 5.8 | 5.8 | 5.8 | 5.8 | 5.8 |
| Phosphoric acid-modified acrylate | 2.5 | 2.5 | 2.5 | 2.5 | 2.5 |
| Dispersant | 1.6 | 1.6 | 1.6 | 1.6 | 1.6 |
| Degree of cure at 10$\mu$m lamination | OK | OK | OK | OK | OK |
| Degree of cure at 50$\mu$m lamination | OK | OK | OK | Uncured | OK |
| Flexural strength (MPa) | 510 | 220 | 360 | 270 | 350 |

The results in Table 2 above are examples prepared by using different types of photoinitiators or varying the content of photoinitiator, and it was confirmed that sufficient degree of cure was secured regardless of the layer thickness, and flexural strength of 200 MPa or more was secured in all of the Examples except for Example 10.

(Table 3)

| | Example 2 | Example 12 | Example 13 | Example 14 |
|---|---|---|---|---|
| 3 mol% Yttria-stabilized zirconia | 75 | 75 | 75 | 75 |
| omnirad 819 | 0.3 | 0.3 | 0.3 | 0.3 |
| THFA (Tetrahydrofufuryl Acrylate monofunctional monomer) | 14.8 | | | |
| HDDA (1,6 Hexanediol diacrylate bifunctional monomer) | | 14.8 | | |
| TMPTA (Tetrahydrofufuryl Acrylate trifunctional monomer) | | | 14.8 | |
| DPHA (Tetrahydrofufuryl Acrylate hexafunctional monomer) | | | | 14.8 |
| Urethane acrylate | 5.8 | 5.8 | 5.8 | 5.8 |
| Phosphoric acid-modified acrylate | 2.5 | 2.5 | 2.5 | 2.5 |
| Dispersant | 1.6 | 1.6 | 1.6 | 1.6 |
| Flexural Strength (MPa) | 510 | 320 | 110 | 60 |

The results in Table 3 above are examples prepared by blending materials with different numbers of monomer functional groups, and it was confirmed that flexural strength of 200 MPa or more was secured in all the Examples except for Examples 13 and 14.

(Table 4)

| | Example 2 | Example 15 | Example 16 | Example 17 | Example 19 |
|---|---|---|---|---|---|
| 3 mol% Yttria-stabilized zirconia | 75 | 75 | 75 | 75 | 75 |
| omnirad 819 | 0.3 | 0.3 | 0.3 | 0.3 | 0.3 |

(continued)

|  | Example 2 | Example 15 | Example 16 | Example 17 | Example 19 |
|---|---|---|---|---|---|
| THFA | 14.8 | 14.8 | 14.8 | 14.8 | 14.8 |
| Urethane acrylate | 5.8 | 5.8 | 5.8 | 3.3 | 8.3 |
| Phosphoric acid-modified acrylate | 2.5 | | | | |
| Carboxylic acid-modified acrylate | | 2.5 | | 2.5 | |
| Epoxy acrylate | | | 2.5 | 2.5 | |
| Dispersant | 1.6 | 1.6 | 1.6 | 1.6 | 1.6 |
| Platform adhesion | OK | OK | OK | OK | NG |
| Flexural Strength (MPa) | 510 | 540 | 500 | 570 | - |

In Examples 1 to 19 described above, the flexural strength is the flexural strength measured after separating the manufactured product from the platform, and washing and sintering it. The washing was performed manually and semi-automatically using alcohol, and degreasing was performed at 500°C for 2 hours, followed by sintering at 1500°C for 2 hours for densification. The results of Table 4 are examples prepared depending on the presence or absence of the use of special oligomers, and it was confirmed that sufficient platform adhesion was secured and at the same time flexural strength of 200 MPa or more was secured in all of the Examples except for Example 19. In particular, in the case of Example 19, it was confirmed that normal output was not performed, and the laminated product was not attached to the platform, but only partially attached to the film of the tank, which corresponded to a defect.

(Table 5)

|  | Example 20 | Example 21 | Example 22 | Example 23 | Example 24 | Example 25 | Example 26 |
|---|---|---|---|---|---|---|---|
| 3 mol% Yttria-stabilized zirconia | 80 | 80 | 80 | 80 | 80 | 80 | 80 |
| omnirad 819 | 0.25 | 0.25 | 0.25 | 0.25 | 0.25 | 0.25 | 0.25 |
| THFA | 12.75 | 12.75 | 12.75 | 12.75 | 12.75 | 12.75 | 12.75 |
| Urethane acrylate | 3.7 | 3.7 | 3.2 | 3.2 | 2.7 | 2.2 | |
| Phosphoric acid-modified acrylate | 1.5 | | 2.0 | | | | 2.6 |
| Carboxylic acid-modified acrylate | | 1.5 | | 2.0 | | | 2.6 |
| Epoxy acrylate | | | | | 2.5 | 3.0 | |
| Dispersant | 1.8 | 1.8 | 1.8 | 1.8 | 1.8 | 1.8 | 1.8 |
| Platform adhesion | NG | NG | OK | OK | NG | OK | OK |
| Flexural Strength (MPa) | - | - | 610 | 600 | | 580 | 650 |

The results of Table 5 above are examples prepared depending on the amount of special oligomer used, and it was confirmed that as in Examples 22, 23, 25 and 26, when the content of the oligomer containing an acidic group in the liquid binder (corresponding to the photocurable binder described above) was 8.5% or more, preferably 10% or more, more preferably 11.14% or more, sufficient platform adhesion was secured, and at the same time, flexural strength of 200 MPa or more was secured. In addition, when the content of the oligomer containing an epoxy group in the liquid binder was 14% or more, preferably 15% or more, and more preferably 16.7% or more, sufficient platform adhesion was secured and at the same time, flexural strength of 200 MPa or more was secured. For example, in the Examples of Table 5, it can be seen that while the platform adhesion was insufficient in Example 20 in which 8.4% of phosphoric acid-modified acrylate was included in the liquid binder, the platform adhesion was excellent in Example 22 in which 11.14% of phosphoric acid-modified acrylate was included in the liquid binder. It can be seen that while the platform adhesion was insufficient in Example 24 in which 13.9% of epoxy acrylate was included in the liquid binder, the platform adhesion was excellent in Example 25 in which 16.7% of epoxy acrylate was included in the liquid binder. The mixing ratio of the special oligomer in the liquid binder can be calculated by the following equation.

(Equation 1)

Mixing ratio of special oligomer in liquid binder = special oligomer content / liquid binder content

The liquid binder content in Equation 1 above may be calculated as the total amount of the binder used to prepare the liquid slurry as in the following equation.

(Equation 2)

Liquid binder content = monomer content + oligomer content

In the case of the Examples of Table 5, the monomer in Equation 2 may be THFA. Further, the oligomer may be urethane acrylate, phosphoric acid-modified acrylate, carboxylic acid-modified acrylate, and epoxy acrylate. Accordingly, Equation 2 above may be used as follows.

(Equation 3)

Liquid binder content = THFA content + urethane acrylate content + phosphoric acid-modified acrylate content + carboxylic acid-modified acrylate content + epoxy acrylate content

The content of the special oligomer in Equation 1 may be determined by the content of the oligomer containing an acidic group and the oligomer containing epoxy. For example, in the case of the Examples of Table 5, the special oligomer content may be calculated as in the following equation.

(Equation 4)

Special oligomer content = phosphoric acid-modified acrylate content + carboxylic acid-modified acrylate content + epoxy acrylate content

Although the present disclosure has been described with reference to the embodiments shown in the drawings, this is only an example, and an ordinary skilled person in the art will understand that various modifications and equivalent other embodiments may be made based thereon. Accordingly, the technical protection scope of the present disclosure should be determined by the technical idea of the accompanying claims.

**INDUSTRIAL APPLICABILITY**

The present specification discloses a method for manufacturing an artificial dental root.

**Claims**

1. An artificial dental root which contains ceramic component and has a plurality of fluid channels formed therein.

2. The artificial dental root of claim 1, comprising: an inner body and an outer body, wherein the fluid channels are formed more in the outer body than in the inner body.

3. The artificial dental root of claim 2, wherein the outer body is formed of a plurality of layers, and
   wherein the fluid channels are formed more in an outermost outer body forming a surface of the artificial dental root than in a first external body in contact with the inner body.

4. The artificial dental root of claim 1, wherein the ceramic component includes tetragonal zirconia, and further includes at least one of alumina, hydroxyapatite, and tricalcium phosphate.

5. The artificial dental root of claim 1, comprising: an inner body and an outer body, wherein the outer body includes at least one of alumina, hydroxyapatite, and tricalcium phosphate more than the inner body.

6. An artificial dental root which contains a ceramic component and comprises at least one of alumina, hydroxyapatite, and tricalcium phosphate.

7. The artificial dental root of claim 6, comprising: an inner body and an outer body,
   wherein the outer body includes at least one of alumina, hydroxyapatite, and tricalcium phosphate more than the inner body.

# FIG. 1

Single-material experimental
artificial dental root

$3Y-ZrO_2$
$ZrO_2-Al_2O_3$
$ZrO_2-HA$
$ZrO_2-TCP$
$ZrO_2-Al_2O_3-HA$
$ZrO_2-Al_2O_3-TCP$
etc.

# FIG. 2

Single-material artificial
dental root with single
structure

$3Y-ZrO_2$
$ZrO_2-Al_2O_3$
$ZrO_2-HA$
$ZrO_2-TCP$
$ZrO_2-Al_2O_3-HA$
$ZrO_2-Al_2O_3-TCP$ etc.

→ Porous structure

# FIG. 3

Dense artificial dental root formed of inner core and gradient functional material

3Y-$ZrO_2$
$ZrO_2$-$Al_2O_3$
$ZrO_2$-HA
$ZrO_2$-TCP
$ZrO_2$-$Al_2O_3$-HA
$ZrO_2$-$Al_2O_3$-TCP etc.

Gradient functional material

# FIG. 4

| | 3Y-ZrO$_2$ | Al$_2$O$_3$ | HAP | TCP | sintering temperature (°C) | relative density (%) | flexural strength (MPa) |
|---|---|---|---|---|---|---|---|
| Z | 100 | 0 | 0 | 0 | 1500 | 99.9 | 857 |
| ZA1 | 90 | 10 | 0 | 0 | 1600 | 99.5 | 580 |
| ZA2 | 80 | 20 | 0 | 0 | 1600 | 99.7 | 620 |
| ZA3 | 70 | 30 | 0 | 0 | 1600 | 99.6 | 596 |
| ZH1 | 90 | 0 | 10 | 0 | 1400 | 99.2 | 538 |
| ZH2 | 80 | 0 | 20 | 0 | 1400 | 99.4 | 513 |
| ZH3 | 70 | 0 | 30 | 0 | 1400 | 99.7 | 427 |
| ZH4 | 60 | 0 | 40 | 0 | 1400 | 99.6 | 249 |
| ZH5 | 50 | 0 | 50 | 0 | 1400 | 99.8 | 124 |

# FIG. 5

Artificial dental root having porous structure and formed of inner core and gradient functional material

$3Y-ZrO_2$
$ZrO_2-Al_2O_3$
$ZrO_2-HA$
$ZrO_2-TCP$
$ZrO_2-Al_2O_3-HA$
$ZrO_2-Al_2O_3-TCP$ etc.

• Gradient functional material

• Porous structure

# FIG. 6

(a)

603 601 602 604

605

Inner core

ZrO₂_HA 10% composite

→ $ZrO_2$ 85%, HA 15%
→ $ZrO_2$ 80%, HA 20%
→ $ZrO_2$ 75%, HA 25%
→ $ZrO_2$ 70%, HA 30%

(b)

603 601 602 604

605

Inner core

$ZrO_2$ Porosity of 0.1% or less

→ Porosity of 5%
→ Porosity of 10%
→ Porosity of 20%
→ Porosity of 30%

EP 4 285 862 A1

# FIG. 7

Liquid photopolymer

UV light

Induced polymerization by light

○ Monomer
◎ Oligomer
◆ Photoinitiator

# FIG. 8

# FIG. 9

# FIG. 10

## INTERNATIONAL SEARCH REPORT

| International application No. |
| --- |
| **PCT/KR2022/001190** |

| A. | CLASSIFICATION OF SUBJECT MATTER |
| --- | --- |

**A61C 8/00**(2006.01)i; **A61L 27/04**(2006.01)i; **A61L 27/40**(2006.01)i; **A61L 27/56**(2006.01)i; **B33Y 80/00**(2015.01)i; **B33Y 10/00**(2015.01)i

According to International Patent Classification (IPC) or to both national classification and IPC

| B. | FIELDS SEARCHED |
| --- | --- |

Minimum documentation searched (classification system followed by classification symbols)

A61C 8/00(2006.01); A61K 6/02(2006.01); A61L 27/00(2006.01); C04B 35/486(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인공 치근(artificial tooth root), 세라믹(ceramic), 채널(channel), 하이드록시 아파타이트(hydroxyapatite, HA), 지르코니아(zirconia), 알루미나(alumina), 트리칼슘포스페이트(tricalcium phosphate), 다공(porous)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | KR 20-0392306 Y1 (AUSTEM CO., LTD.) 19 August 2005 (2005-08-19)<br>See paragraph [0042]; claims 1 and 3-4; and figure 3. | 1-2,5 |
| Y |  | 3-4 |
| Y | JP 5166864 B2 (HI-LEX CORP. INC.) 21 March 2013 (2013-03-21)<br>See paragraphs [0027], [0105] and [0107]; and figure 14. | 3 |
| X | JP 3287952 B2 (KYOCERA CORP.) 04 June 2002 (2002-06-04)<br>See paragraphs [0009]-[0011] and [0013]; claim 1; and figures 2-3. | 6-7 |
| Y |  | 4 |
| X | JP 07-213540 A (NISHIHARA, K. et al.) 15 August 1995 (1995-08-15)<br>See claims 1 and 4-8. | 1,6 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **26 April 2022** | **27 April 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2022/001190** |

| C. | DOCUMENTS CONSIDERED TO BE RELEVANT | |
| --- | --- | --- |
| Category* | Citation of document, with indication, where appropriate, of the relevant passages | Relevant to claim No. |
| X | KR 10-2015-0034177 A (IVOCLAR VIVADENT AG) 02 April 2015 (2015-04-02)<br>See claim 19. | 1 |

International application No.

**PCT/KR2022/001190**

| Patent document cited in search report | | | Publication date (day/month/year) | Patent family member(s) | | | Publication date (day/month/year) |
|---|---|---|---|---|---|---|---|
| KR | 20-0392306 | Y1 | 19 August 2005 | None | | | |
| JP | 5166864 | B2 | 21 March 2013 | CN | 101141987 | A | 12 March 2008 |
| | | | | CN | 101141987 | B | 19 September 2012 |
| | | | | CN | 101920046 | A | 22 December 2010 |
| | | | | EP | 1852135 | A1 | 07 November 2007 |
| | | | | EP | 1852135 | B1 | 11 April 2018 |
| | | | | JP | 2006-263445 | A | 05 October 2006 |
| | | | | JP | 2006-263458 | A | 05 October 2006 |
| | | | | US | 2009-0215009 | A1 | 27 August 2009 |
| | | | | WO | 2006-090777 | A1 | 31 August 2006 |
| | | | | WO | 2006-090777 | A1 | 24 July 2008 |
| JP | 3287952 | B2 | 04 June 2002 | JP | 07-323038 | A | 12 December 1995 |
| JP | 07-213540 | A | 15 August 1995 | JP | 2547953 | B2 | 30 October 1996 |
| | | | | US | 5584693 | A | 17 December 1996 |
| KR | 10-2015-0034177 | A | 02 April 2015 | CN | 104470871 | A | 25 March 2015 |
| | | | | CN | 108439979 | A | 24 August 2018 |
| | | | | EP | 2877438 | A2 | 03 June 2015 |
| | | | | EP | 2877438 | B1 | 27 February 2019 |
| | | | | JP | 2015-529609 | A | 08 October 2015 |
| | | | | JP | 6063040 | B2 | 18 January 2017 |
| | | | | PT | 2877438 | T | 06 June 2019 |
| | | | | US | 10315958 | B2 | 11 June 2019 |
| | | | | US | 11040914 | B2 | 22 June 2021 |
| | | | | US | 2015-0191397 | A1 | 09 July 2015 |
| | | | | US | 2019-0248709 | A1 | 15 August 2019 |
| | | | | WO | 2013-190043 | A2 | 27 December 2013 |
| | | | | WO | 2013-190043 | A3 | 20 March 2014 |